# EUROPEAN PATENT APPLICATION

(11) **EP 1 913 935 A1**
(43) Date of publication of application: **23.04.2008**
(21) Application number: 07020984.6
(22) Date of filing: 20.01.2006
(51) Int. Cl.: A61K 9/16, A61K 9/48, A61K 31/423, A61K 9/14

(54) **Stable pharmaceutical formulations of zonisamide and methods for their manufacture**

(30) Priority: 21.01.2005 US 645030 P
(62) Divisional of application: 06250301.6
(71) Applicant: Teva Pharmaceutical Industries Ltd, 49131 Petah Tiqva (IL)
(72) Inventor: Hrakovsky, Julia, Rosh Ha-Ayin 48057 (IL); Tenengauzer, Ruth, Hod Hasharon 45322 (IL)
(74) Representative: Gallagher, Kirk James

(57) **Abstract**

The present invention is directed toward a process for preparing a stable zonisamide pharmaceutical composition, comprising subjecting zonisamide to wet granulation with a granulation liquid to form a granulated mixture as the stable zonisamide pharmaceutical composition, wherein the granulation liquid is selected from purified water, alcohol and mixtures thereof. The stable zonisamide pharmaceutical composition can be used to fill capsule shells to prepare stable zonisamide capsules.

## Description

### FIELD OF THE INVENTION

The present invention relates to a stable pharmaceutical composition comprising zonisamide as the active ingredient which may be prepared by subjecting zonisamide to wet granulation with a granulation liquid, and methods of preparing the stable pharmaceutical composition. The invention also concerns stable pharmaceutical capsules containing the stable pharmaceutical composition comprising zonisamide and methods of preparing the capsules.

### BACKGROUND OF THE INVENTION

Zonisamide is known as 1,2-benzisoxazole-3-methane sulfonamide or 3-(sulfamoylmethyl)-1,2-benzisoxazole. It is currently available, in capsules, as an anti-epileptic agent which possesses anti-convulsant and anti-neurotoxic effects.

Zonisamide capsules in the prior art can be prepared by a conventional process of mixing powders of zonisamide and suitable pharmaceutical excipients followed by filling hard gelatin capsule shells with the mixed powders to form the capsules. However, zonisamide capsules prepared by the prior art process suffer at least two disadvantages. First, the powders inside the prior art zonisamide capsules become lumpy upon storage of the capsules. Second, as a result of the lumpy powders, the release rate of zonisamide measured by in vitro dissolution studies is reduced.

There is a need to improve on the preparation of pharmaceutical capsules containing zonisamide as the active ingredient. The present invention overcomes the problems encountered by prior art zonisamide capsules by providing novel stable zonisamide capsules prepared by methods exemplified by at least two novel methods for preparing zonisamide pharmaceutical formulations. The zonisamide capsules of the invention prepared by either one of the novel methods or similar methods are stable in that no lumps are formed with the ingredients inside the capsules upon storage, resulting in stable dissolution rates.

### SUMMARY OF THE INVENTION

One of the embodiments of the present invention provides a novel stable zonisamide pharmaceutical composition which may be prepared by a method comprising subjecting zonisamide to wet granulation with a granulation liquid, e.g. purified water, alcohol and mixtures thereof, to form a granulated mixture as the stable zonisamide pharmaceutical composition. The stable zonisamide pharmaceutical composition may contain one or more pharmaceutically acceptable excipients such as diluents, binders, disintegrants and lubricants mixed with zonisamide and the granulation liquid. The stable zonisamide pharmaceutical composition can be used to fill capsule shells to obtain stable zonisamide capsules.

Within the scope of the invention is the method of preparing the novel stable zonisamide pharmaceutical composition, comprising subjecting zonisamide to wet granulation with the granulation liquid to form the stable zonisamide pharmaceutical composition in the form of a granulated mixture, wherein the method optionally further comprises mixing one or more pharmaceutically acceptable excipients such as diluents, binders, disintegrants and lubricants with zonisamide and the granulation liquid. The method may further comprise filling capsule shells with the stable zonisamide pharmaceutical composition.

Another embodiment of the present invention is directed toward a novel stable zonisamide pharmaceutical composition comprising a capsule shell filled with an intimate mixture, wherein the intimate mixture comprises zonisamide and an effective amount of at least one pharmaceutically acceptable excipient, such as a pharmaceutically acceptable carbohydrate, wetting agent and glidant or combination of two or more thereof. The invention also provides a method for preparing the novel stable zonisamide pharmaceutical composition, which method comprises:
(i) forming an intimate mixture of components by compressing, co-milling, co-micronizing and/or co-compacting the components, and/or by subjecting the components to one or more similarly intensive processes, wherein the components comprise zonisamide powder and an effective amount of at least one pharmaceutically acceptable excipient, such as a carbohydrate, wetting agent, glidant and/or combination of two or more thereof; and
(ii) filling capsule shells with the intimate mixture to obtain a stable zonisamide pharmaceutical composition.

Alternatively, in the method for preparing the stable zonisamide pharmaceutical composition via the intimate mixture, one or more pharmaceutical excipients are mixed with the intimate mixture formed in step (i) to obtain a combination and then the capsule shells are filled with the combination in step (ii), wherein the "one or more pharmaceutical excipients" are one or more pharmaceutical excipients the same as or different from the at least one pharmaceutical excipient used in step (i) to form the intimate mixture. For instance, the "one or more pharmaceutical excipients" can be the pharmaceutically acceptable wetting agent and/or glidant used in the intimate mixture, or pharmaceutical acceptable excipients other than the pharmaceutically acceptable wetting agent and/or glidant.

The present invention includes the stable zonisamide pharmaceutical composition which can be in the form of the stable zonisamide pharmaceutical capsule prepared by any of the methods described in the present patent application.

### DETAILED DESCRIPTION OF THE INVENTION

In the present patent application, when a pharmaceutical composition or formulation is described as stable, the term 'stable" means that the pharmaceutical composition or formulation has a dissolution rate that does not decrease, when subjected to accelerated storage conditions of 40° C at 75% relative humidity for 1, 2 and 3 months, by more than 10%, preferably by more than 5%, of the initial dissolution rate. Preferably, the solid ingredients in the "stable" pharmaceutical composition or formulation of the invention do not form lumps when subjected to accelerated storage conditions of 40° C at 75% relative humidity for 1, 2 and 3 months.

In the present invention dissolution rate may be measured using the following methodology: USP apparatus II (paddle), 75 rpm, 900 ml water at 37° C, sampling time 45 minutes and analysis with a UV detector at 284 nm.

Whether a pharmaceutical composition or formulation of the present invention forms lumps upon storage at accelerated conditions may be determined visually or via sieving analysis. If the composition or formulation is subjected to sieving analysis, the following procedure is performed. First, the smallest screen-mesh at least 99.5% of the composition or formulation is able to pass through is determined with a sample of the composition or formulation before storage at 40°C and 75% relative humidity. The smallest screen-mesh is hereinafter referred to as the "initial minimal screen-mesh." Another sample of the composition or formulation previously stored at 40°C and 75% relative humidity for 1, 2 or 3 months is then sieved with the initial minimal screen-mesh. If more than 97%, preferably more than 99%, of the post-storage sample is able to pass through the initial minimal screen-mesh, the composition or formulation is described as being lump free.

Within the scope of the invention is a stable zonisamide pharmaceutical composition prepared by a method comprising
(a) mixing zonisamide powder with a granulation liquid and optionally at least one pharmaceutically acceptable excipient selected from the group consisting of pharmaceutically acceptable diluents, binders, disintegrants and lubricants, wherein the granulation liquid is selected from purified water, alcohol and mixtures thereof, to form a wet powder blend; and
(b) milling the wet and/or dry powder blend,
wherein there is a drying step either between step (a) and step (b), or after step (b), to form the stable zonisamide pharmaceutical composition. The present invention also includes the method for preparing the stable zonisamide pharmaceutical composition.

The method optionally further comprises, after step (a), sifting the wet powder blend through a screen to remove or break up any lumps to form screened wet granules, wherein the screened wet granules are subjected to drying either before or after step (b) to form the stable zonisamide pharmaceutical composition. One or more excipients may be added to the dry granules. Optionally, the method further comprises filling capsule shells with the stable zonisamide pharmaceutical composition to form stable zonisamide capsules.

In the method for preparing the stable zonisamide pharmaceutical composition via the intimate mixture, the effective amount of the at least one pharmaceutically acceptable excipient, such as the carbohydrate, wetting agent and/or glidant, in the intimate mixture in step (i) can be about 0.1 wt% to about 10 wt% of each wetting agent or glidant individually when the at least one pharmaceutically acceptable excipient includes one or more wetting agents and/or glidants, and about 1 wt% to about 90 wt% of all carbohydrates combined when the at least one pharmaceutically acceptable excipient includes one or more carbohydrates, wherein the wt% is based on the total weight of the intimate mixture. For instance, when the at least one pharmaceutical acceptable excipient in the intimate mixture comprises a pharmaceutically acceptable wetting agent, pharmaceutically acceptable glidant and pharmaceutically acceptable carbohydrate, the intimate mixture can contain, for instance, in addition to the zonisamide powder, about 0.1 wt% to about 10 wt% of the wetting agent, about 0.1 wt% to about 10 wt% of the glidant and about 1 wt% to about 90 wt% of the carbohydrate. When the at least one pharmaceutical acceptable excipient in the intimate mixture comprises a pharmaceutically acceptable glidant and two pharmaceutically acceptable carbohydrates, the intimate mixture can contain, in addition to the zonisamide powder, about 0.1 wt% to about 10 wt% of the glidant and about 1 wt% to about 90 wt% of the carbohydrates combined.

The pharmaceutically acceptable wetting agent can be selected from pharmaceutically acceptable surfactants, more preferably pharmaceutically acceptable anionic surfactants such as alkyl sulfates, e.g. sodium lauryl sulfate, sodium stearyl sulfate, sodium oleyl sulfate and sodium cetyl sulfate, alkyl aryl sulfonates, e.g. sodium dodecylbenzene sulfonate and dialkyl sodium sulfosuccinates, e.g. sodium bis-(2-ethylhexyl)sulfosuccinate, and most preferably sodium lauryl sulfate. Further examples of the pharmaceutically acceptable wetting agent include benzethonium chloride, cetylpyridinium chloride, docusate sodium, poloxamer, polysorbate and sorbitan esters. The pharmaceutically acceptable glidant can be selected from talc, calcium stearate, calcium phosphate tribasic, calcium silicate, powdered cellulose, magnesium silicate, magnesium trisilicate, starch and, preferably, colloidal silicon dioxide. The pharmaceutically acceptable excipient used to make the intimate mixture with zonisamide powder can be selected from pharmaceutically acceptable carbohydrates, such as sucrose, mannitol, sorbitol, lactose and glucose, in solid form. Other examples of the pharmaceutically acceptable excipient are microcrystalline cellulose and hydroxypropyl cellulose.

The stable zonisamide pharmaceutical composition in the form of a capsule prepared by any of the methods described in the present patent application can contain 25, 50 or 100 mg zonisamide per capsule.

One of the objects of the present invention is to provide a solid pharmaceutical formulation, e.g. in the form of capsules, which releases from about 60% to about 100% of zonisamide as the active ingredient in about 45 minutes at release in an aqueous environment. The release rate of the pharmaceutical formulation is maintained during the shelf life.

Another object of the present invention is drawn to a solid dosage form of zonisamide comprising an intimate mixture, which intimate mixture comprises solid zonisamide powder and at least one pharmaceutically acceptable excipient, wherein the solid dosage form is stable such that the dissolution rate measured at a sampling time of 45 minutes, of the solid dosage form does not decrease by more than 10% than its initial dissolution rate upon storage at 40° C and 75% relative humidity for up to three months, when tested by the parameters described herein. Preferably, the solid dosage form is also stable in that no lumps are formed upon storage at 40° C and 75% relative humidity for up to three months.

In the present patent application, the term "intimate mixture" means a mixture more intimate than normal powder mixes, e.g., powder mixes prepared by dry mixing or blending of zonisamide powder with one or more pharmaceutical acceptable excipients. The term "intimate mixture" refers to a mixture of closely-packed components. The intimate mixture may be produced by co-milling, co-micronization and/or co-compaction of the components and similarly intensive processes.

Without being bound by any theory on how the present invention works, it is believed that the intimate mixture can render the zonisamide pharmaceutical composition or formulation stable because the at least one pharmaceutically acceptable excipient in the intimate mixture prevents the zonisamide powder to adhere to each other to form lumps over time with the at least one pharmaceutically acceptable excipient coating the zonisamide powder and separating the zonisamide powder from each other. By preventing the formation of lumps over time upon storage, the dissolution rate of the zonisamide pharmaceutical composition or formulation made from the intimate mixture can be maintained at a value that does not change more than 10% over time upon storage even for up to three months at accelerated conditions.

Some of the embodiments of the present invention are illustrated with the working examples below, which are for demonstration purposes. One skilled in the art can practice the invention beyond the scope of the working examples based on the disclosures in the present patent application.

### EXAMPLES

### Comparative Example 1-3

The following batches of a pharmaceutical composition containing zonisamide were prepared by a dry mix method. The ingredients of Part I were blended for 15 minutes to form an initial blend. Part II ingredients were added to the initial blend and the final blend was encapsulated into capsules. The Part I ingredients and Part II ingredients used are shown in Table 1.

**Table 1**

| Ingredient | Comp. Ex. 1 | Comp. Ex. 2 | Comp. Ex. 3 |
|---|---|---|---|
| | *K-33094^{*}* | *K-33395^{*}* | *K-33396^{*}* |
| | (mg/capsule) | (mg/capsule) | (mg/capsule) |
| Part I: | | | |
| Zonisamide | 100.0 | 100.0 | 100.0 |
| Microcrystalline cellulose | 170.0 | 170.0 | 140.0 |
| Colloidal silicon dioxide | 1.5 | 1.5 | 1.5 |
| Sodium lauryl sulfate | 1.5 | 1.5 | 1.5 |
| | | | |

| Part II: | | | |
|---|---|---|---|
| Microcrystalline cellulose | 55.5 | 55.5 | 45.5 |
| Colloidal silicon dioxide | 1.5 | 1.5 | 1.5 |
| Hydrogenated vegetable oil | 10.0 | 10.0 | 10.0 |
| | | | |
| Total | 340.0 | 340.0 | 300.0 |
| Capsule Shell No. | No.1 | No.0 | No.0 |

| | | | |
|---|---|---|---|
| ^{*}Batch number | | | |

### Working Example 1

The ingredients of Part I of Table 2 were granulated using a purified water as a granulating liquid. The granulate was dried, milled and blended with a Part II ingredient. The final blend was encapsulated into capsules. The ingredients of Part I and Part II used are shown in Table 2.

**Table 2**

| Ingredient | *K-34327*^{*} |
|---|---|
| | (mg/capsule) |
| Part I: | |
| Zonisamide | 207.9 |
| Microcrystalline cellulose | 100.0 |
| Sodium lauryl sulfate | 1.5 |
| Crospovidone | 20.0 |
| Povidone | 5.0 |
| | |

| Part II: | |
|---|---|
| Hydrogenated vegetable oil | 5.6 |
| | |
| Total | 340.0 |
| Capsule Shell No. | No.0 |

| | |
|---|---|
| *Batch number | |

### Working Example 2

Preparation of zonisamide capsule by dry mixing using an intimate admixture -

Part I materials were milled through 0.8 mm Frewitt oscillating granulator, blended with Part II materials and encapsulated into capsules. The materials of Parts I and II used are shown in Table 3.

**Table 3**

| Ingredient | *K-34393^{*}* |
|---|---|
| | (mg/capsule) |
| Part I: | |
| Zonisamide | 100.0 |
| Colloidal silicon dioxide | 3.0 |
| Sodium lauryl sulfate | 3.0 |
| | |

| Part II: | |
|---|---|
| Microcrystalline cellulose | 224.0 |
| Hydrogenated vegetable oil | 10.0 |
| | |
| Total | 340.0 |
| Capsule Shell No. | No.0 |

| | |
|---|---|
| *Batch number | |

### Properties of Zonisamide Capsules of Comparative Examples and Working Examples

A stability test was performed on samples of Comparative Examples 1-3 (each batch in Table 1) initially after they were prepared and at various times after storage at accelerated conditions, i.e., 40° C and 75% relative humidity, for 1 or 2 months. The results are shown in Table 4.

Dissolution method: USP apparatus II (paddle), 75 rpm, 900 ml water at 37° C, sampling time 45 minutes, analysis using UV detector at 284 nm.

**Table 4.**

| Batch No. | Initial | 1 mon. 40°C/75% RH | 2 mon. 40°C/75% RH |
|---|---|---|---|
| *K-33094* | Avg. 94% | Avg. 74% | Avg.91% |
| | Mini.^{*} 88% | Mini.^{*} 45% | Mini.^{*} 80% |
| | | | |
| *K-33395* | | Avg. 80% | Avg. 76% |
| | | Mini.^{*} 55% | Mini.^{*} 51% |
| | | | |
| *K-33396* | | Avg. 91% | Avg. 86% |
| | | Mini.^{*} 87% | Mini.^{*} 60% |

| | | | |
|---|---|---|---|
| ^{*}Minimum | | | |

The stability results generated on the zonisamide capsules of Comparative Examples 1-3 demonstrated reduction of the dissolution rates upon storage at accelerated conditions. Visual inspections of the zonisamide capsules of Comparative Examples 1-3 indicated that these capsules contained lumps of ingredients.

The water content of the zonisamide capsule of Comparative Example I was determined by the Karl Fischer method. The Karl Fischer results showed that the water content of the zonisamide capsule was maintained at the same level during storage at 40° C and 75% relative humidity, or 25° C and 60% relative humidity (Table 5).

**Table 5**

| | | Karl Fischer Results | | | |
|---|---|---|---|---|---|
| Batch No. | Initial | 1 mon., 40°C, 75% RH | 2 mon., 40°C, 75% RH | 3 mon., 40°C, 75% RH | 3 mon., 25°C, 60% RH |
| K-33094 | 3.4 | 3.6% | 4.0% | 4.0% | 3.7% |

A stability test was performed on samples of Working Examples 1 and 2 (each batch in Tables 2 and 3) initially after they were prepared and at various times after storage at accelerated conditions, i.e., 40° C and 75% relative humidity, for 1, 2 or 3 months. The results are shown in Table 6.

Dissolution method: USP apparatus II (paddle), 75 rpm, 900ml water at 37° C, sampling time 45 minutes, analysis using UV detector at 284 nm.

**Table 6.**

| Batch No. | Initial | 1 mon. 40° C 75% RH | 2 mon. 40° C 75% RH | 3 mon. 40° C 75% RH |
|---|---|---|---|---|
| *K-34327* | Avg. 95% | Avg. 88% | Avg. 94% | Avg. 96% |
| | Mini.^{*} 90% | Mini.^{*} 78% | Mini.^{*} 91% | Mini.^{*} 92% |
| | | | | |
| *K-34393* | Avg. 93% | Avg. 96% | Avg. 97% | Avg. 94% |
| | Mini.^{*} 90% | Mini.^{*} 93% | Mini.^{*} 95% | Mini.^{*} 90% |

| | | | | |
|---|---|---|---|---|
| ^{*}Minimum | | | | |

Visual inspections of the zonisamide capsules of Working Examples 1 and 2 did not show any lumps. The zonisamide capsules also proved to be stable. The dissolution results demonstrated that the zonisamide capsules of Working Examples 1 (K-34327) and 2 (K-34393) were stable.

Further aspects and features of the present invention are set out in the following numbered clauses.
1. A stable pharmaceutical dosage form comprising, in intimate mixture, solid particulate zonisamide and at least one pharmaceutically acceptable excipient.
2. The stable pharmaceutical dosage form of clause 1, wherein no lumps are formed in the dosage form upon storage at 40° C and 75% relative humidity for up to three months.
3. The stable pharmaceutical dosage form of clause 1 or clause 2, wherein the dissolution rate of the zonisamide from the solid dosage form in an aqueous environment over 45 minutes does not decrease by more than 10% from its initial dissolution rate upon storage at 40° C and 75% relative humidity for up to three months.
4. The stable pharmaceutical dosage form of any preceding clause, wherein the at least one pharmaceutically acceptable excipient is selected from pharmaceutically acceptable wetting agents, pharmaceutically acceptable glidants, pharmaceutically acceptable carbohydrates and combination of two or more thereof.
5. The stable pharmaceutical dosage form of clause 4, wherein the at least one pharmaceutically acceptable wetting agent is selected from alkyl sulfates, alkyl aryl sulfonates and dialkyl sodium sulfosuccinates.
6. The stable pharmaceutical dosage form of clause 4, wherein the at least one pharmaceutically acceptable wetting agent is selected from the group consisting of sodium lauryl sulfate, sodium stearyl sulfate, sodium oleyl sulfate, sodium cetyl sulfate, sodium dodecylbenzene sulfonate, sodium bis-(2-ethylhexyl)sulfosuccinate, benzethonium chloride, cetylpyridinium chloride, docusate sodium, poloxamer, polysorbate and sorbitan esters.
7. The stable pharmaceutical dosage form of clause 6, wherein the at least one pharmaceutically acceptable excipient comprises sodium lauryl sulfate.
8. The stable pharmaceutical dosage form of clause 4; wherein the at least one pharmaceutically acceptable glidant is selected from talc, calcium stearate, calcium phosphate tribasic, calcium silicate, powdered cellulose, magnesium silicate, magnesium trisilicate, starch and colloidal silicon dioxide.
9. The stable pharmaceutical dosage form of clause 8, wherein the at least one pharmaceutically acceptable excipient comprises colloidal silicon dioxide.
10. The stable pharmaceutical dosage form of clause 4, wherein the at least one pharmaceutically acceptable carbohydrate is selected from sucrose, glucose, lactose, mannitol and sorbitol.
11. A process for preparing a stable zonisamide pharmaceutical composition, comprising subjecting zonisamide to wet granulation with a granulation liquid to form a granulated mixture as the stable zonisamide pharmaceutical composition, wherein the granulation liquid is selected from purified water, alcohol and mixtures thereof.
12. The process of clause 11, further comprising adding one or more pharmaceutically acceptable excipients in or after the wet granulation step.
13. The process of clause 12, wherein the one or more pharmaceutically acceptable excipients are selected from pharmaceutically acceptable diluents, binders, disintegrants and lubricants.
14. The process of clause 12 or 13, further comprising filling capsule shells with the granulated mixture.
15. The process of any of clauses 11 to 14, wherein the wet granulation step is performed by mixing a zonisamide powder with the granulation liquid to form a powder blend; and milling the powder blend.
16. The process of clause 15, further comprising sifting the powder blend through a screen to remove or break up any lumps after the wet granulation step.
17. The process of clause 15 or 16, further comprising drying the powder blend between the mixing and milling steps, between the milling step and the sifting step or after the sifting step.
18. A process for preparing a stable zonisamide pharmaceutical capsule, comprising
   (i) forming an intimate mixture of components, wherein the components comprises zonisamide powder and an effective amount of at least one pharmaceutically acceptable excipient; and
   (ii) filling capsule shells with the intimate mixture to obtain the stable zonisamide pharmaceutical capsule.
19. The process of clause 18, wherein the at least one pharmaceutically acceptable excipient in step (i) is selected from pharmaceutically acceptable carbohydrates, pharmaceutically acceptable wetting agents, pharmaceutically acceptable glidants and combination of two or more thereof.
20. The process of clause 18 or 19, wherein the effective amount of the at least one pharmaceutically acceptable excipient is about 0.1 wt% to about 10 wt% of a wetting agent or glidant individually when the at least one pharmaceutically acceptable excipient includes one or more wetting agents and/or glidants, and about 1 wt% to about 90 wt% of one or more carbohydrates combined when the at least one pharmaceutically acceptable excipient includes one or more carbohydrates, wherein the wt% is based on the total weight of the intimate mixture.
21. The process of clause 18 or 19, wherein the at least one pharmaceutically acceptable excipient comprises at least one pharmaceutically acceptable wetting agent
22. The process of clause 21, wherein the at least one pharmaceutically acceptable wetting agent is selected from pharmaceutically acceptable surfactants.
23. The process of clause 22, wherein the pharmaceutically acceptable surfactants are selected from alkyl sulfates, alkyl aryl sulfonates and dialkyl sodium sulfosuccinates.
24. The process of clause 22 or 23, wherein the at least one pharmaceutically acceptable wetting agent is selected from the group consisting of sodium lauryl sulfate, sodium stearyl sulfate, sodium oleyl sulfate, sodium cetyl sulfate, sodium dodecylbenzene sulfonate, sodium bis-(2-ethylhexyl)sulfosuccinate, benzethonium chloride, cetylpyridinium chloride, docusate sodium, poloxamer, polysorbate and sorbitan esters.
25. The process of clause 24, wherein the at least one pharmaceutically acceptable excipient comprises sodium lauryl sulfate.
26. The process of any of clauses 18 to 20, wherein the at least one pharmaceutically acceptable excipient comprises at least one pharmaceutically acceptable glidant.
27. The process of clause 26, wherein the at least one pharmaceutically acceptable glidant is selected from talc, calcium stearate, calcium phosphate tribasic, calcium silicate, powdered cellulose, magnesium silicate, magnesium trisilicate, starch and colloidal silicon dioxide.
28. The process of clause 27, wherein the at least one pharmaceutically acceptable excipient comprises colloidal silicon dioxide.
29. The process of any of clauses 18 to 28, wherein one or more additional pharmaceutical acceptable excipients are mixed with the intimate mixture between step (i) and step (ii) to obtain a combination, wherein in step (ii) the capsule shells are filled with the combination to form the capsule.
30. The process of clause 29, wherein the one or more pharmaceutical acceptable excipients are one or more pharmaceutical excipients other than the at least one pharmaceutically acceptable excipient included in the intimate mixture of step (i).
31. The process of clause 30, wherein the one or more pharmaceutical acceptable excipients are selected from pharmaceutically acceptable diluents, binders and disintegrants.
32. The process of any of clauses 18 to 31, wherein the intimate mixture is formed by compressing, co-milling, co-micronizing and/or co-compacting the components.
33. A stable zonisamide pharmaceutical composition prepared by the process of any of clauses 11 to 32.

## Claims

1. A process for preparing a stable zonisamide pharmaceutical composition, comprising subjecting zonisamide to wet granulation with a granulation liquid to form a granulated mixture as the stable zonisamide pharmaceutical composition, wherein the granulation liquid is selected from purified water, alcohol and mixtures thereof.

2. The process of claim 1, further comprising adding one or more pharmaceutically acceptable excipients in or after the wet granulation step.

3. The process of claim 2, wherein the one or more pharmaceutically acceptable excipients are selected from pharmaceutically acceptable diluents, binders, disintegrants and lubricants.

4. The process of claim 2 or 3, further comprising filling capsule shells with the granulated mixture.

5. The process of any of claims 1 to 4, wherein the wet granulation step is performed by mixing a zonisamide powder with the granulation liquid to form a powder blend; and milling the powder blend.

6. The process of claim 5, further comprising sifting the powder blend through a screen to remove or break up any lumps after the wet granulation step.

7. The process of claim 5 or 6, further comprising drying the powder blend between the mixing and milling steps, between the milling step and the sifting step or after the sifting step.

8. A stable zonisamide pharmaceutical composition prepared by the process of claim 11.
